# EUROPÄISCHE PATENTSCHRIFT

(11) **EP 1 738 781 B1**
(45) Veröffentlichungstag und Bekanntmachung des Hinweises auf die Patenterteilung: **16.03.2011**
(21) Anmeldenummer: 06011581.3
(22) Anmeldetag: 03.06.2006
(51) Int. Cl.: A61L 27/36, A61L 27/54, A61L 31/00, A61L 31/16

(54) **Verwendung von Propolis als Beschichtungmaterial für medizinische Implantate**
Use of propolis for coating medical implants
Utilisation de propolis pour revêtir des implants médicaux

(30) Priorität: 30.06.2005 DE 102005031361
(43) Veröffentlichungstag der Anmeldung: 03.01.2007
(73) Patentinhaber: Biotronik VI Patent AG, 6341 Baar (CH)
(72) Erfinder: Hesselbarth, Rudolf, 79774 Albbruck (DE); Rohde, Roland, 31303 Burgdorf (DE)
(74) Vertreter: Lindner-Vogt, Karin L.

(56) Entgegenhaltungen:
- WO-A-99/49830
- DE-A1- 10 350 654
- RO-B1- 113 205
- RU-C1- 2 106 781
- US-A- 4 382 886

## Beschreibung

Die Erfindung betrifft eine neue Verwendung von Propolis als Beschichtungsmaterial für medizinische Implantate zur Behandlung von Erkrankungen des Herz-Kreislauf-Systems wie Schrittmacher, Defibrillatoren, Herz- oder Venenklappen, Gefäßstützen oder Gefäßprothesen. Sie betrifft weiterhin die Verwendung von Propolis, insbesondere des Inhaltsstoffs Kaffeesäurephenylethylester zur Herstellung eines Arzneimittels zur Restenose-Prophylaxe nach perkutaner transluminaler koronarer Angioplastie (PTCA) in Koronararterien.

Permanente oder für den zeitweiligen Verbleib im Körper ausgelegte medizinische Implantate erfordern zur Minimierung von unerwünschten Gewebereaktionen nach Implantation ein hohes Maß an Biokompatibilität der verwendeten Werkstoffe. Mittlerweile kann auf eine Vielzahl unterschiedlichster biokompatibler Materialien zurückgegriffen werden. Im Bereich von Gefäßstützen (Stents) kann beispielsweise eine Biokompatibilität der Implantatsoberfläche dadurch verbessert werden, dass die Implantatsoberfläche mit Siliziumcarbid oder Phosphorylcholin beschichtet ist, was die Thrombogenität und etwaige Entzündungsreaktion an der Implantatsoberfläche mindert.

Ein weiterer Problempunkt moderner Implantationstechnik liegt in dem Umstand, dass es immer wieder zu einer bakteriellen Besiedlung des Implantats kommt, wobei nicht notwendigerweise jede Besiedlung zu einer Infektion führen muss. Zunächst wird das Implantat mit körpereigenen Proteinen, wie Fibrin, Fibronectin, Albumin, Laminin oder Vitronectin bedeckt, an die sich Bakterien (wie z. B. *staphylococcus aureus*) anheften können. Die Bakterien synthetisieren Exopolysaccharide und bilden einen Biofilm auf der Oberfläche des Implantats. Die heterogene adhärente Bakterienpopulation des Biofilms ist für Abwehrmechanismen des Körpers sowie antibiotische Therapien nur schwer zugänglich. *Staphylococcus epidermis* wurde als häufigster Mikroorganismus bei Infektionen von Implantaten, insbesondere von intravaskulären Katheter-Infektionen und Schrittmacher/Defibrillator-Infektionen, isoliert. *Staphylococcus aureus* und koagulase-negative Staphylokokken sind weitere häufig auftretende Bakterien bei Implantatinfektionen. Derartige Infektionen können häufig nur durch Explantation und erneute Implantation, ggf. begleitend mit systemischer Antibiotikagabe behandelt werden. Beispielsweise wird bei Infektionen von Schrittmacher- oder Schrittmacher-Defibrillator-Sytemen eine systemische Antibiotikagabe mit einer chirurgischen Behandlung empfohlen.

Da es sehr schwierig ist, die Matrix des Biofilms mittels Antibiotika zu durchdringen und die mit dem Biofilm assoziierten Zellen abzutöten, müssen Strategien entwickelt werden, die entweder die Polysaccharidmatrix des Biofilms gezielt angreifen oder die Anheftung der Bakterien und damit die Bildung eines Biofilms verhindern. Letztere Alternative sieht beispielsweise die Aufbringung einer Beschichtung auf dem Implantat vor, die entweder aus sich selbst heraus antibiotisch wirkt oder Antibiotika enthält. So wurden beispielsweise Herzklappen-Nahtstellen mit Silber oder Katheter mit Silbersalzen oder Antibiotika beschichtet.

Hat man sich für ein Beschichtungssystem zur Verbesserung der Biokompatibilität und/oder Minderung der Infektionsgefahr entschieden, so ist die Auswahl eines geeigneten Beschichtungsmaterials alles andere als trivial, denn das Material sollte sich nach Möglichkeit mit herkömmlichen Fertigungstechniken in einfacher Weise verarbeiten lassen, eine möglichst gleichmäßige Oberflächenabdeckung des Implantats erlauben, natürlich biokompatibel und antiinfektiös sein, in ausreichenden Mengen verfügbar sein und nach Möglichkeit natürlich auch kostengünstig sein. Allein das Auffinden eines geeigneten Werkstoffs erfordert daher ein hohes Maß an Verständnis der zu Grunde liegenden biologischen Mechanismen, die Kenntnis der gewünschten Materialeigenschaften hinsichtlich der Verarbeitung und späteren Verwendung und eben auch Kenntnisse über die Verfügbarkeit und die möglichen Kosten, die mit dem Einsatz des Werkstoffs verbunden sind. Das Auffinden eines solchen Materials ist demnach sehr aufwendig und kann nicht in standardisierter Weise erfolgen, gerade auch deshalb, weil viele Materialeigenschaften, die für den Verwendungszweck eine Rolle spielen können, noch nicht beschrieben oder nicht voraussehbar sind und erst in aufwendigen Versuchen belegt werden müssen.

Trotz der in den letzten Jahrzehnten enormen Bemühungen zur Verbesserung der angesprochenen Probleme in der Implantattechnik und der fortschreitenden Aufklärung der zu Grunde liegenden biologischen Mechanismen, der ständigen Weiterentwicklung und Neuentdeckung geeigneter Werkstoffe und dem Einsatz komplexer Beschichtungssysteme zur Minderung von Abstoßungsreaktionen und Infektionen, besteht nach wie vor ein hoher Bedarf an neuen Lösungsansätzen, die die Situation verbessern oder zumindest Alternativen zu Bekanntem bieten. Die Veröffentlichung RO113205 offenbart ein Dentalimplantat, das einen zylindrischen Abschnitt aus einem speziellen Tannenbaummaterial, aus Kokosfasern oder anderen speziellen Holzsorten besteht. Dieser Zylinder ist in eine Lösung oder einem Destillat von Propolis getaucht/imprägniert.

Das in RO113205 genannte Implantat erfüllt jedoch nicht die an Implantate zur Behandlung von Erkrankungen des Herz-Kreislauf-Systems geforderte hohe Biokompatibilität und die Vermeidung von Infektionsgefahr.

Der Erfindung liegt die Aufgabe zu Grunde, die Biokompatibilität von medizinischen Implantaten zur Behandlung von Erkrankungen des Herz-Kreislauf-Systems zu verbessern und eine Infektionsgefahr zu Vermeiden sowie ein Arzneimittel zur Restenose-Prophylaxe bereitszustellen.

Diese Aufgabe wird durch die Verwendung von Propolis zur Herstellung a) eines Beschichtungsmaterials für medizinische Implantate zur Behandlung von Erkrankungen des Herz-Kreislauf-Systems und b) zur Herstellung eines Arzneimittels zur Restenose-Prophylaxe gelöst.

Propolis ist eine dunkelgelbliche bis hellbraune, harzartige, zwischen den Fingern erweichende Masse mit würzig-balsamartigem Geruch und einer Schmelztemperatur zwischen 50 - 70°C, die von Bienen gesammelt und im Bienenstock als Überzug der Wände und zum Befestigen der Waben benutzt wird (Vorwachs, Stopfwachs, Bienenleim, Bienenkitt, Bienenharz). Propolis ist ein Naturstoff dessen qualitative und quantitative Zusammensetzung stark variiert. Folgende Inhaltsstoffe wurden bereits isoliert und beschrieben

### a) Aminosäuren

In geringen Mengen enthält Propolis Pyroglutaminsäure, die vermutlich aus dem Bienen-Metabolismus stammt

### b) Aliphatische Säuren und ihre Ester

Es wurden langkettige Säuren (z. B. Behen-, Palmitin-, Stearin- und Myristinsäure) in Propolis gefunden, die ihren Ursprung wahrscheinlich im Bienenwachs haben. Der Ursprung der ebenfalls vorhandenen kurzkettigen, unbeständigen Säuren, wie Succin-, Angelica- und Buttersäure sowie ihren Estern (z. B. Isobuttersäure) ist unbekannt, aber vermutlich pflanzlichem Ursprungs.

### c) Aromatische Säuren und deren Ester

Die aromatischen Säuren und deren Ester sind zumeist pflanzlichen Ursprungs. Wichtige Beispiele sind Zimtsäure, Methylsalicylat, Kaffeesäure und ihre Ester, Vanillinsäure und p-Cumarsäurebenzaldehyd.

### d) Alkohole

Einige Glycerinderivate entstammen höchstwahrscheinlich dem Bienen-Metabolismus und Glycerol kommt im Wachs vor. Andere Alkohole, wie z. B. Hydrochinon und Zimtalkohol, finden sich auch in Pappeln.

### e) Aldehyde

Die wichtigen Vertreter der Aldehyde sind Vanillin, Protocatechualdehyd (3,4-Dihydroxybenzaldehyd), p-Hydroxybenzaldehyd.

### f) Chalkone

Chalkone zählen zu den Flavonoiden und akkumulieren als Glykoside nur in wenigen Pflanzenfamilien. Das in der Natur am häufigsten auftretende Chalkon ist Butein (2',3,4,4'-Tetrahydroxychalkon).

### g) Dihydrochalkone

Dihydrochalkone sind Flavanonglykoside und z. B. charakteristisch für Propolis balsamifera (Sektion Tacamhaca) und kommen nur in geringen Mengen in Propolis vor.

### h) Flavanone, Flavane und Flavonole

Flavanone sind den phenolischen Verbindungen zugeordnete Derivate des Flavans (Phenyl-4H-chroman), die in 4-Stellung eine Oxo-Gruppe besitzen und auch als hydrierte Flavone aufgefasst werden können. Flavonole sind zu den Flavanoiden zählende Klasse von im Allgemeinen gelben, cremefarbenen, geruch- und geschmacklosen Pflanzenfarbstoffen, denen das - bei den Flavonolen in 3-Stellung hydroxylierte - Grundgerüst des Flavons gemeinsam ist. Flavane sind als hydrierte Flavone aufzufassen. Die Verbindungen stellen eine Hauptkomponente von europäischen Propolis dar. Hauptvertreter sind Pinocembrin (5,7-Dihydroxyflavanon), Pinobanksin (3,5,7-trihydroxy-flavanonol), Naringenin, Sakuranetin, Galangin (3,5,7-Trihydroxyflavon), Quercetin (3,3',4',5,7-Pentahydroxflavon).

### i) Kohlenwasserstoffe, Ketone, Terpenoide und andere Komponenten

Ketone, Terpenoide und andere Komponenten sind nur in geringen Mengen in Propolis vorhanden. Den C₁₀-Terpenoiden wird ein strenger Geruch nachgesagt und sie sind wahrscheinlich für den Duft von Propolis verantwortlich.

Die in Propolis vorhandenen erheblichen Wachsanteile erwiesen sich Analysen zu Folge in ihren Hauptbestandteilen als Monoester und Kohlenwasserstoffe. Die Kohlenwasserstoffe bestehen aus einem komplexen Gemisch von n-Alkanen, die eine ungerade Anzahl von C-Atomen im Bereich von C₂₃ - C₃₅ besitzen. Am häufigsten wurde C₂₇H₅₆, C₂₉H₆₀, C₃₁H₄₆ und C₃₃H₆₈ gefunden.

Propolis für die erfindungsgemäßen Zwecke wird wie folgt standardisiert: Es werden Propolis-Proben aus gemäßigten Klimazonen, vorzugsweise Propolis-Proben von Pappeln des Sektion Aigeiros, gesammelt und zusammengeführt, deren Konsistenz zunächst entsprechend den obigen Angaben anhand von Farbe und Geruch überprüft wird. Ggf. vorhandene Feststoffanteile (z. B. Holz) werden mechanisch entfernt. Ergänzend erfolgt eine chemisch-analytische Bestimmung des Wachsanteils, der zwischen 10 - 30 Gew. % liegen soll.

In einer bevorzugten Ausführungsform umfasst der Begriff Propolis vorliegend auch ein aus dem zuvor geschilderten standardisierten Propolis durch Aufarbeitung erhaltenes aufbereitetes Propolis. Ein Wachsanteil des aufbereiteten Propolis liegt vorzugsweise zwischen 15 - 25 Gew. %. Die Aufbereitung erfolgt vorzugsweise unter Einsatz herkömmlicher Verfahren zur Abtrennung von ggf. vorhandenen Eiweißstoffen oder anderen möglichen Allergenen. Weiterhin ist bevorzugt, wenn der aufbereitete Propolis einem Sterilisationsverfahren unterzogen wird, bei dem beispielsweise durch Zufuhr thermischer Energie leicht flüchtige Bestandteile ausgetrieben und chemische Reaktionen innerhalb des Propolis stattfinden könnten, die eine Zusammensetzung des Produktes ändern.

Als aufbereitete Propolis im Sinne der Erfindung wird auch ein zur Beschichtung geeignetes Gemisch verstanden, das Propolis in einem Gewichtsanteil von mindestens 30 Gew.%, vorzugsweise mindestens 50 Gew.% und besonders bevorzugt mindestens 80 Gew.% enthält. Die weiteren Bestandteile eines derartigen Gemisches können beispielsweise Bienenwachs, Fette, Kohlenwasserstoffe, Fettsäuren oder dergleichen sein, deren Zusatz zur Vereinfachung der Verarbeitung des Materials sinnvoll sein kann oder eine verbesserte Anpassung der Materialeigenschaften an ein spezifisches Implantat bewirken kann. So ist beispielsweise denkbar, durch Zusatz langkettiger Verbindungen, wie Wachsen oder Fetten, die Viskosität und das Haftvermögen des Materials zu beeinflussen.

Denkbar und bevorzugt ist auch, Propolis als Trägermatrix für pharmazeutische Wirkstoffe, wie beispielsweise Paclitaxel oder Sirolimus, zu nutzen. Hier ist vorteilhaft, das Propolis hydrophob ist und somit schlecht wasserlösliche pharmazeutische Wirkstoffe in größeren Mengen lösen kann. Nach Implantation werden die Wirkstoffe durch Diffusion allmählich in das umliegende Gewebe gelangen und ihre bestimmungsgemäße Wirkung entfalten.

Es hat sich gezeigt, dass Propolis antibakterielle, antifungale, antivirale, tumorcytotoxische, tumorhemmende, lokalanästhetische, antiinflammatorische und spasmolytische Eigenschaften hat. Einige aktive Komponenten der Propolis, denen die vorgenannten Wirkungen zugeschrieben werden, konnten bereits identifiziert werden. So wird (i) Pinocembrin, Galangin, Kaffeesäure und Ferulasäure eine antibakterielle Wirkung (ii) Pinocembrin, 3-Acetylpinobanksin, Kaffeesäure, p-Cumarsäurebenzylester, Sakuranetin, Pterostilben eine antifungale Wirkung (iii) Kaffeesäure, Quercetin eine antivirale Wirkung (iv) Kaffeesäurephenylethylester (CAPE) eine tumorcytotoxische oder tumorhemmende Wirkung (v) Pinocembrin, Pinostrobin, Kaffeesäureester eine lokalanästhetische Wirkung (vi) Kaffeesäure eine antiinflammatorische Wirkung und (vii) Quercetin, Kaempferid, Pectolinaringenin eine spasmolytische Wirkung zugeschrieben.

Propolis ist leicht zugänglich, zu geringen Kosten käuflich erhältlich, bei Körpertemperatur von klebriger, wachsartiger Konsistenz und lässt sich gut mit herkömmlichen Verfahrenstechniken in gleichmäßiger Bedeckung auf Implantatsoberflächen aufbringen.

Die genannten Wirkungen und Eigenschaften prädestinieren Propolis als Beschichtungsmaterial für medizinische Implantate.

Gerade Erkrankungen des Herz-Kreislauf-Systems bergen die Gefahr, dass Infektionen außer Kontrolle geraten, zumal bei der Implantation von Schrittmachern und Defibrillatoren den bereits obig beschriebenen Abläufen durch allmähliche Biofilmbildung auf dem Implantat Vorschub geleistet wird und die Patienten aufgrund der Erkrankung schon geschwächt sind. Schrittmacher und Defibrillatoren sind komplexe elektrische Geräte, die üblicherweise nach Außen durch eine Schale abgegrenzt sind. Herzklappen oder Venenklappen sind filigrane Gebilde, die zur Gewährung Ihrer Funktionalität eine Vielzahl von Bedingungen erfüllen müssen. Bei einer Gefäßprothese handelt es sich um einen Schlauch, der ein Blutgefäß - meistens eine Arterie - ersetzt oder überbrückt. Eine Geometrie der Implantate und die für die Schalen / Klappen / Schläuche gewählten Werkstoffe sind nicht ohne weiteres zur Minderung eines Abstoßungs- bzw. Infektionsrisikos veränderlich. Weiterhin ist eine Explantation von Schrittmachern und Defibrillatoren oder Herz-und Veneklappen oder Gefäßprothesen für den Patienten auf Grund des Krankheitsbildes besonders belastend. Aus diesem Grunde bietet sich für Schrittmacher und Defibrillatoren, Herz- und Veneklappen oder Gefäßprothesen eine Beschichtung mit Propolis besonders an.

Herauszuheben als Implantate zur Behandlung von Erkrankungen des Herz-Kreislauf-Systems, welche besonders für die Verwendung von Propolis zum Einsatz als Beschichtungsmaterial geeignet sind, sind Gefäßstützen.

Bei 70% aller perkutanen Interventionen werden Gefäßstützen (Stents) eingesetzt, in 25% aller Fälle kommt es jedoch zu einer in-Stent Restenose mit einem überschießenden neointimalen Wachstum, das durch eine starke Proliferation der arteriellen glatten Muskelzellen und eine chronische Entzündungsreaktion hervorgerufen wird. Zur Senkung der Restenoseraten wurden die verschiedensten Ansätze verfolgt, wie z. B. eine intrakoronare radioaktive Bestrahlung (Brachytherapie), bei der jedoch Rand-Restenosen, eine verzögerte Heilung und eine unvollständige Endothelialisierung auftraten. In den letzten Jahren wurden Stents mit diversen pharmazeutischen Wirkstoffen beschichtet, entweder durch direkte Anbindung an die Stentoberfläche oder Einbettung in ein Polymer als Trägermatrix. Dabei ist eine ausreichend lokale Wirkstoffkonzentration und -verteilung in der Gefäßwand notwendigerweise erstrebenswert. Am erfolgreichsten bei der Senkung der Restenoserate waren bisher die Wirkstoffe Sirolimus oder Paclitaxel freisetzende Stents. Es treten in jüngster Zeit jedoch Fälle subakuter Thrombosen und allergische Reaktionen auf, die vermutlich durch die für die Trägermatrix verwendeten Polymere hervorgerufen werden. Besonders Patienten mit multipler oder schwieriger Symptomatik (wie Diabetes, komplexe Läsionen, kleine Gefäße, lange Läsionen) zeigen teils eine erhöhte Thromboserate. Für die Stentbeschichtung (die Trägermatrix) können nicht abbaubare Polymere (z. B. Polyurethane, Polymethacrylate), degradierbare Polymere (z. B. Polyhydroxybuttersäure, Polylactide), synthetische Polymere (Phosphorylcholin) oder Polymere biologischen Ursprungs (Hyaluronsäure) verwendet werden. Ein Teil der Polymere ruft starke Entzündungsreaktionen hervor oder induziert eine unerwünschte Proliferation. Daher wird zu einer direkten Anbindung des Wirkstoffs an die Stentoberfläche ohne Polymer übergegangen, jedoch ist diese technisch aufwendig. Durch letztere Maßnahme kann zudem nur die Gefahr einer Restenose nach perkutaner transluminaler koronarer Angioplastie (PTCA) gemindert oder bestenfalls ausgeräumt werden. Die Gefahr von Infektionen ist damit nicht gebannt und kann bei Stents auf Grund des schnellen Einwachsens in das umliegende Gewebe nur noch systemisch behandelt werden. Hier kann ein Beschichtungsmaterial, das sowohl antiinfektiöse Eigenschaften besitzt, aber auch nach ersten Erkenntnissen der Anmelderin zur Restenose-Prophylaxe geeignet ist, Abhilfe schaffen. Daher ist Propolis besonders geeignet als Beschichtungsmaterial für Gefäßstützen.

Nach einer bevorzugten Ausführungsform besteht die Gefäßstütze ganz oder in Teilen aus einer biokorrodierbaren metallischen Legierung, insbesondere Magnesiumlegierung. Biokorrodierbar bedeutet, dass der Werkstoff nach Implantation allmählich, z. B. durch hydrolytische oder enzymatische Prozesse, abgebaut wird. Derartige Legierungen sind beispielsweise aus DE 1 419 793 A1 bekannt, deren Offenbarungsgehalt bezüglich der verwendeten Magnesiumlegierungen vollumfänglich hiermit einbezogen wird. Der Einsatz von Propolis als Beschichtungsmaterial für Gefäßstützen aus einer biokorrodierbaren metallischen Legierung, insbesondere Magnesiumlegierung, ist deshalb besonders bevorzugt, weil Propolis bekanntlich hydrophob ist und damit eine Beschichtung des Implantats die Abbauprozesse hemmt/verzögert. Mit anderen Worten, durch eine derartige hydrophobe Beschichtung kann das Degradationsverhalten des Implantats gesteuert werden. Gegebenenfalls kann beispielsweise durch Vorgabe unterschiedlicher Beschichtungsdicken in unterschiedlichen Bereichen des Implantats das Degradationsverhalten gezielt an einzelnen Punkten des Implantates beeinflusst und so z. B. eine möglichst gleichmäßige Degradation des Implantats erreicht werden.

Ein weiterer Aspekt der Erfindung liegt in der überraschenden Erkenntnis, dass Propolis sich zur Herstellung eines Arzneimittels zur Restenose-Prophylaxe in humanen Koronararterien nach perkutaner transluminaler koronarer Angioplastie (PTCA) eignet.

Nach ersten Versuchen der Anmelderin zeigte sich insbesondere, dass der in Propolis enthaltene Kaffeesäurephenylethylester (CAPE; CAS 104594-70-9) der Formel (I) 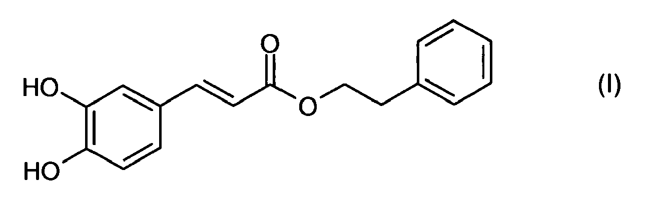 vermutlich einer der aktiven Komponenten ist und zur Restenose-Prophylaxe in humanen Koronararterien nach perkutaner transluminaler koronarer Angioplastie (PTCA) geeignet ist.

In diesem Zusammenhang ist bekannt, dass Kaffeesäurephenylethylester ein Inhibitor des Transkriptionsfaktors NFκB ist (nachgewiesen u. a. (i) für humane Brustkrebszellen des Typ MCF-7: M. Watabe et al., The Journal of Biological Chemistry, Vol. 279, No. 7, pp. 6017 - 6026, 2004 und (ii) für T-Zellen: N. Märquez et al., The Journal of Pharmacology and Experimental Therapeutics, Vol. 308, No. 3, pp. 999 - 1001, 2004). Weiterhin beschreibt K. Yamasaki et al. in Gene Therapy, Vol. 10, pp. 356 - 364, 2003 Versuche am Schweinemodell, bei denen eine Neointimalbildung durch Applikation eines cis-Element Decoys zur Inhibition von NFκB nach Ballonangioplastie deutlich reduziert ist.

Überraschend war es nun, dass Propolis die Proliferation humaner arterieller glatter Muskelzellen aus Koronararterien zu 75% im Vergleich zu unbehandelten Zellen inhibierte, während die Vitalität und Proliferation von humanen arteriellen Endothelzellen bei der gleichen Konzentration der Substanz nur um 30% reduziert wird. Hinsichtlich der neointimalen Proliferation ist eine Inhibition der glatten Muskelzellen erwünscht. Das Wachstum der Endothelzellen ist hingegen für eine Endothelialisierung wichtig. Versuche mit humanen arteriellen Endothelzellen zur Inhibition der TNFα induzierten NFκB-Aktivierung zeigten eine nahezu vollständige Hemmung der NFκB-Aktivierung durch Propolis (gemessen als Translokation von der p65 Untereinheit im Kern; Nachweis mit Immunfluoreszenz). Nach derzeitigem Kenntnisstand wird davon ausgegangen, dass einer der an diesem zelltypspezifischen Wirkmechanismus beteiligten Inhaltsstoffe der Propolis Kaffeesäurephenylethylester ist. Propolis und die genannte Verbindung eignen sich daher besonders für eine lokale Therapie der durch PTCA betroffenen koronaren Gefäßabschnitte durch Aufbringung derselben als Beschichtungsmaterial auf Gefäßprothesen oder Kathetern.

Es folgt eine kurze Beschreibung einiger Zellversuche.

### Verwendete Zellen

Humane arterielle Endothelzellen (EC) und humane arterielle glatte Muskelzellen (SMC), kommerziell erhältlich bei PromoCell GmbH, Heidelberg, Deutschland.

### Verwendete Kulturmedien

"Endothelial Cell Growth Medium MV Kit" und "Smooth Muscle Cell Growth Medium 2 Kit", kommerziell erhältlich bei PromoCell GmbH, Heidelberg, Deutschland.

### Kultivierung der Zellen

Die Durchführung erfolgte nach den in der Zellkulturtechnik gebräuchlichen Standardvorschriften und nach den Arbeitsanweisungen von Promocell.

### Eingesetzte Materialien

Propolis-Rohmaterial (Hersteller: R. Hesselbarth, Albbruck-Buch, Deutschland; Beschaffenheit: Gelblich-braune, harzig-wachsartige Substanz mit Koniferen-geruch) wurde in 70%-igem Ethylalkohol gelöst. Nach Absetzen der unlöslichen Bestandteile wurde der Überstand filtriert und in fest verschließbaren Glasbehältern aufbewahrt. Der Feststoffgehalt des für die Kulturexperimente verwendeten Extraktes wurde zu 450mg Trockensubstanz pro ml Extrakt bestimmt.

### Versuchsvorbereitung / Zellkultivierung

Zunächst wurden Verdünnungsreihen des Propolisextraktes (unter Einsatz einer 70%-igen wässrigen Ethanollösung als Verdünnungsmedium) hergestellt und später den Zellkulturen in solcher Menge zugegeben, dass Konzentrationen von 16,37 µg/ml, 8,175 µg/ml, 4,1 µg/ml und 2,05µg/ml Trockenmasse pro ml Zellkulturansatz resultierten. Kontrollproben des Lösungsmittels wurden zwecks Bezugs auf einen eigenständigen Einfluss des Lösungsmittels auf Proliferation und Vitalität der Zellen mitgeführt.

Zur in vitro Testung der Substanzen wurden 48 Stunden nach Aussaat der zu testenden Zellen (96-well Platten, 0,3x10⁴ Zellen/well) den Kulturen die oben genannten verdünnten Propolisansätze zugegeben und unmittelbar nach den unten genannten Inkubationszeiten die Vitalitäts- und Proliferationstests (siehe unten) nach den entsprechenden Arbeitsvorschriften der Testhersteller durchgeführt.

In den einzelnen Experimenten wurde Propolis in drei parallelen Ansätzen getestet.

### Vitalitätstest (MTS)

Nach Inkubation der Zellen zu den unterschiedlichen Inkubationszeiten (12h, 24h, 48h, 72h bei 37°C und 5% CO₂), wurde die Vitalität gemessen. Dabei wurde der "CellTiter 96 Aqueous One Solution Cell Proliferation Assay" der Firma Promega GmbH, Deutschland verwendet. Dieser Test ist eine colorimetrische Methode zur Bestimmung der Stoffwechselaktivität von Zellen.

### Proliferationstest (BrdU-ELISA)

Die Proliferation der Zellen wurde nach den unterschiedlichen Inkubationszeiten (12h, 24h, 48h, 72h bei 37°C und 5% CO₂) gemessen. Dazu wurde der "Cell Proliferation Elisa BrdU" von Roche Deutschland Holding GmbH, Deutschland verwendet. Die Messung wurde mit dem "µQuant" Elisa-Reader von MWG Biotech AG, Deutschland durchgeführt.

### Aktivierung von NFκB

Primäre humane arterielle EC bzw. SMC wurden in 24-well Platten ausgesät (1,5x104/well). Einen Tag später wurden die Zellen mit den verschiedenen Verdünnungen von Propolis (16,35 µg/ml, 8,175 µg/ml, 4,1 µg/ml, 2,05 µg/ml Zellkulturansatz) für eine Stunde und 40 Minuten inkubiert. Anschließend erfolgte eine weitere Inkubation für 45 Minuten mit bzw. ohne 25 ng/ml TNFα (Sigma-Aldrich Chemie GmbH, Deutschland). Als Positiv- und Negativ-Kontrolle wurden die Zellen nur mit bzw. ohne 25 ng/ml TNFα inkubiert. Anschließend wurden die Zellen mit PBS gewaschen, 20 Minuten mit 3% PFA inkubiert, TX-100 permeabilisiert ((i) Sodeik B, Ebersold MW, Helenius A.: Microtubulemediated transport of incoming herpes simplex virus 1 capsids to the nucleus. J Cell Biol. 1997 Mar 10; 136(5): 1007 - 1021; (ii) Dohner K, Wolfstein A, Prank U, Echeverri C, Dujardin D, Vallee R, Sodeik B.: Function of dynein and dynactin in herpes simplex virus capsid transport. Mol Biol Cell. 2002 Aug; 13(8): 2795 - 2809) und mit einem Antikörper gegen NFκB p65 (Zymed) markiert. Der Nachweis erfolgte über die Bindung eines Alexa546-gekoppelten sekundär Antikörpers (Molecular Probes) und fluoreszenzmikroskopischer Auswertung.

### Detektion und Apoptose

Primäre humane arterielle EC bzw. SMC wurden in 24-well Platten ausgesät (1,5x10⁴/well). Einen Tag später wurden die Zellen mit verschiedenen Verdünnungen von Propolis (16.37 µg/ml, 8,175 µg/ml, 4,1 µg/ml, 2,05µg/ml) für weitere 24h inkubiert. Danach wurden die Zellen mit PBS gewaschen, 20 Minuten mit 3% PFA inkubiert und mit 0,1% TritonX-100 permeabilisiert. Im Anschluss daran wurde zur Detektion apoptotischer Zellen der TUNEL-Assay nach dem Protokoll von Roche "In Situ Cell Death Detection Kit" durchgeführt. Zum Schluss wurde mit Dapi gegengefärbt. Die Auswertung erfolgte mittels Fluoreszenzmikroskopie.

### Ergebnisse

### Einfluss von Propolis auf die Vitalität von EC und SMC

Untersucht wurde die Wirkung von Propolis in Konzentrationen von 16.37 µg/ml, 8,175 µg/ml, 4,1 µg/ml und 2,05µg/ml Zellkulturansatz. In Vorversuchen hatte sich gezeigt, dass höhere Konzentrationen sowohl für die colorimetrischen Assays auf Grund der intensiven Eigenfarbe von Propolis als auch durch die hohe Zelltod-Induzierbarkeit nicht geeignet waren.

Die Vitalität der humanen arteriellen glatten Muskelzellen (SMC) wie auch der Endothelzellen (EC) ist nicht nur abhängig von der Konzentration von Propolis, sondern auch von der Dauer der Inkubationszeit. Je höher die Konzentration von Propolis, desto stärker wird ebenfalls die Vitalität der Zellen gehemmt. Dabei ist die Hemmung der Vitalität in den ersten 24h am stärksten; danach ist eine Abschwächung der Inhibition zu beobachten. Zudem konnte festgestellt werden, dass die EC gegenüber Propolis unempfindlicher sind als die SMC. Bei gleicher Konzentration und Inkubationszeit war die Vitalität der EC bei Inkubation mit Propolis um etwa die Hälfte bis ein Drittel weniger gehemmt als die Vitalität der SMC.

### Einfluss von Propolis auf die Proliferation von EC und SMC

Untersucht wurde die Wirkung von Propolis in Konzentrationen von 16,37 µg/ml, 8,175 µg/ml, 4,1 µg/ml und 2,05µg/ml. In Vorversuchen hatte sich ebenfalls gezeigt, dass höhere Konzentrationen sowohl für die colorimetrischen Assays auf Grund der intensiven Eigenfarbe von Propolis als auch durch die hohe Zelltod-Induzierbarkeit nicht geeignet waren.

Der Einfluss von Propolis auf die Proliferation von SMC und die EC ist wie der Einfluss auf die Vitalität sowohl konzentrationsabhängig als auch von der Dauer der Inkubationszeit abhängig. Je höher die Konzentration von Propolis und je länger die Inkubationszeit, desto stärker wird die Proliferation der Zellen gehemmt.

Ebenso wie bei der Vitalität ist die Hemmung der Proliferation in den ersten 24h am stärksten; danach wird die Hemmung schwächer.

Hierbei zeigt sich ebenfalls eine geringere Sensibilität der EC gegenüber Propolis im Vergleich zu den SMC. Bei gleicher Konzentration und Inkubationszeit war die Proliferation der EC durch Propolis um ein Vielfaches geringer gehemmt als die Proliferation der SMC.

### Untersuchung auf die Aktivierung von NFκB

Die EC zeigt sich eine nahezu vollständige Hemmung der TNFα indizierten NFκB-Aktivierung durch Propolis (1:32). Höhere Verdünnungen hatten keinen inhibitorischen Effekt auf die TNFα induzierte NFκB-Aktivierung. Um auszuschließen, dass Propolis selbst NFκB aktiviert, wurden die Zellen mit Propolis ohne TNFα inkubiert. Bei allen untersuchten Konzentrationen zeigte sich keine Aktivierung von NFκB.

### Detektion apoptotischer Zellen

Bei glatten Muskelzellen konnte nach der Zugabe von Propolis in einer Konzentration 8,175 µg/ml ein erster Hinweis auf Apoptose festgestellt werden. Bei der Zugabe von 4,1 µg/ml Propolis konnten aufgrund zu schwacher Signale apoptotische Zellen nicht eindeutig detektiert werden. Bei der geringsten Konzentration 2,05 µg/ml Propolis waren keine apoptischen Zellen zu beobachten.

Bei Endothelzellen konnte bei keiner der verwendeten Konzentrationen eine Apoptose nachgewiesen werden.

## Patentansprüche

1. Verwendung von Propolis zur Herstellung eines Beschichtungsmaterials für medizinische Implantate zur Behandlung von Erkrankungen des Herz-Kreislauf-Systems.

2. Verwendung nach Anspruch 1, **dadurch gekennzeichnet, dass** das medizinische Implantat eine Gefäßstütze ist.

3. Verwendung nach Anspruch 1, **dadurch gekennzeichnet, dass** das medizinische Implantat ein Schrittmacher oder ein Defibrillator ist.

4. Verwendung nach Anspruch 1, **dadurch gekennzeichnet, dass** das medizinische Implantat eine Herz- oder Venenklappe ist.

5. Verwendung nach Anspruch 1, **dadurch gekennzeichnet, dass** das medizinische Implantat eine Gefäßprothese ist.

6. Medizinisches Implantat zur Behandlung von Erkrankungen des Herz-Kreislauf-Systems mit einer Beschichtung bestehend aus oder enthaltend Propolis.

7. Verwendung von Propolis zur Herstellung eines Arzneimittels zur Restenose-Prophylaxe nach perkutaner transluminaler koronarer Angioplastie (PTCA) in humanen Koronararterien.

8. Verwendung von Kaffeesäurephenylethylester zur Herstellung eines Arzneimittels zur Restenose-Prophylaxe nach perkutaner transluminaler koronarer Angioplastie (PTCA) in humanen Koronararterien.

## Claims

1. The application of propolis to manufacture a coating material for medical implants for treating diseases of the cardiovascular system.

2. The application according to claim 1, **characterized in that** the medical implant is a vascular stent.

3. The application according to claim 1, **characterized in that** the medical implant is a pacemaker or a defibrillator.

4. The application according to claim 1, **characterized in that** the medical implant is a heart or venous valve.

5. The application according to claim 1, **characterized in that** the medical implant is a vascular prosthesis.

6. A medical implant for treating diseases of the cardiovascular system, comprising a coating composed of or containing propolis.

7. The use of propolis for the manufacture of a drug for restenosis prophylaxis after percutaneous transluminal coronary angioplasty (PTCA) in human coronary arteries.

8. The use of caffeic acid phenethyl ester for the manufacture of a drug for restenosis prophylaxis after percutaneous transluminal coronary angioplasty (PTCA) in human coronary arteries.

## Revendications

1. Utilisation de propolis pour la fabrication d'un matériau de revêtement pour implants médicaux destinés au traitement de maladies du système cardiovasculaire.

2. Utilisation selon la revendication 1, **caractérisée en ce que** l'implant médical est un renfort vasculaire.

3. Utilisation selon la revendication 1, **caractérisée en ce que** l'implant médical est un stimulateur cardiaque ou un défibrillateur.

4. Utilisation selon la revendication 1, **caractérisée en ce que** l'implant médical est une valvule cardiaque ou veineuse.

5. Utilisation selon la revendication 1, **caractérisée en ce que** l'implant médical est une prothèse vasculaire.

6. Implant médical pour le traitement de maladies du système cardio-vasculaire, avec un revêtement constitué de propolis ou contenant de la propolis.

7. Utilisation de propolis pour la fabrication d'un médicament destiné à la prévention de la restenose, suite à une angioplastie coronaire transluminale percutanée (ACTP) dans les artères coronaires chez l'humain.

8. Utilisation de phényle-éthyle-ester d'acide caféique pour la fabrication d'un médicament destiné à la prévention de la restenose, suite à une angioplastie coronaire transluminale percutanée (ACTP) dans les artères coronaires chez l'humain.
